(19) Europäisches Patentamt European Patent Office Office européen des brevets

(11) **EP 3 079 797 B1**

(12) **FASCICULE DE BREVET EUROPEEN**

(45) Date de publication et mention de la délivrance du brevet:
**15.08.2018 Bulletin 2018/33**

(21) Numéro de dépôt: **14827479.8**

(22) Date de dépôt: **09.12.2014**

(51) Int Cl.:
*C07C 67/08* [(2006.01)]      *B01D 61/36* [(2006.01)]
*C07C 69/54* [(2006.01)]      *C07C 67/56* [(2006.01)]
*B01D 71/02* [(2006.01)]      *B01D 71/38* [(2006.01)]

(86) Numéro de dépôt international:
**PCT/FR2014/053223**

(87) Numéro de publication internationale:
**WO 2015/086978 (18.06.2015 Gazette 2015/24)**

(54) **PURIFICATION D´ESTERS (METH)ACRYLIQUES PAR DÉSHYDRATATION PAR SÉPARATION MEMBRANAIRE**

REINIGUNG VON (METH)ACRYLSÄUREESTERN DURCH MEMBRANTRENNUNGSDEHYDRIERUNG

PURIFICATION OF (METH)ACRYLIC ESTERS BY MEMBRANE SEPARATION DEHYDRATION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.12.2013 FR 1362560**

(43) Date de publication de la demande:
**19.10.2016 Bulletin 2016/42**

(73) Titulaire: **Arkema France**
**92700 Colombes (FR)**

(72) Inventeurs:
• **TRETJAK, Serge**
**F-57520 Roulhing (FR)**

• **MORELIERE, Anne**
**F-57740 Longeville-les-St-Avold (FR)**

(74) Mandataire: **Bonnel, Claudine**
**ARKEMA France**
**Département Propriété Industrielle**
**420, rue d'Estienne d'Orves**
**92705 Colombes Cedex (FR)**

(56) Documents cités:
WO-A1-2004/063140    FR-A1- 2 980 475
GB-A- 770 551        JP-A- 2002 047 213

**Description**

DOMAINE DE L'INVENTION

La présente invention concerne la production d'esters (méth)acryliques, et a plus particulièrement pour objet un perfectionnement à la production de (méth)acrylates d'alkyle en $C_4$ - $C_{10}$, consistant en la mise en oeuvre d'une étape de déshydratation par séparation membranaire d'au moins un flux généré au cours de la purification dudit (méth)acrylate d'alkyle.

ARRIERE-PLAN TECHNIQUE

Il est connu de produire des esters (méth)acryliques à partir d'une réaction d'estérification entre un alcool et un acide (méth)acrylique. Cette réaction est une réaction catalysée équilibrée avec génération d'eau. Elle s'accompagne par ailleurs de réactions secondaires produisant des impuretés.

Il est nécessaire d'éliminer l'eau produite pour déplacer l'équilibre, de recycler les réactifs non réagis (l'alcool et l'acide), ainsi que d'éliminer les impuretés, en particulier les composés plus légers que l'ester et les composés plus lourds que l'ester, de façon à obtenir un produit répondant à des spécifications commerciales.

A cette fin, on procède généralement à un ensemble de traitement du mélange réactionnel à l'aide de distillations et/ou d'extractions, décantations, ensemble qui est à la fois relativement complexe à mettre en oeuvre, notamment du fait de la présence de mélanges azéotropes, et coûteux sur le plan énergétique.

Le mélange réactionnel contient l'ester recherché, de l'eau, de l'acide et de l'alcool non réagis, des sous-produits dits « légers » présentant une température d'ébullition inférieure à celle de l'ester et des sous-produits dits « lourds », c'est-à-dire présentant une température d'ébullition supérieure à celle de l'ester. Le train de purification appliqué au mélange réactionnel génère différents flux dont la composition varie suivant le caractère apolaire de l'alcool et de l'ester, c'est-à-dire selon la longueur de la chaine alkyle de l'alcool utilisé. Ces flux ont en commun de contenir de l'eau issue de la réaction et/ou des étapes d'extraction.

Dans le procédé de production d'ester (méth)acrylique décrit dans la demande de brevet FR 2 980 475 au nom de la Demanderesse, des modules de déshydratation par séparation membranaire sont utilisés pour déshydrater des flux comprenant de l'ester (méth)acrylique et de l'alcool non réagi en vue d'éliminer efficacement l'eau et conduire à une bonne sélectivité en ester (méth)acrylique. Ces modules sont notamment appliqués au niveau de l'étape réactionnelle, ou au niveau du recyclage de l'alcool n'ayant pas réagi pour une gamme de concentration des différents composés bien définie, et principalement dans le cas de la production de (méth)acrylates d'alkyle de $C_1$ à $C_4$, tels que l'acrylate de méthyle, l'acrylate d'éthyle ou l'acrylate de butyle.

De façon surprenante, la Demanderesse a maintenant constaté que dans le cas de la production de (méth)acrylates d'alkyle en $C_4$-$C_{10}$, il est possible d'appliquer une étape de déshydratation par séparation membranaire à des flux aqueux en dehors des gammes de concentration décrites dans le document FR 2 980 475. Cela conduit à l'élimination efficace de l'eau présente dans des flux destinés à être purifiés et/ou recyclés, distincts des flux aqueux traités dans le procédé du document précité, et évite ainsi la formation d'une boucle d'eau néfaste pour la productivité et la consommation énergétique du procédé. En particulier, l'étape de déshydratation est avantageusement appliquée en amont de la colonne de finition de l'ester recherché, ou en tête de la colonne de séparation des composés légers, ou sur la phase aqueuse après décantation du mélange réactionnel brut.

RESUME DE L'INVENTION

L'invention a donc pour objet un procédé de production d'un (méth)acrylate d'alkyle comportant une chaine alkyle linéaire ou ramifiée comportant de 4 à 10 atomes de carbone, par estérification directe de l'acide (méth)acrylique avec un alcool linéaire ou ramifié comportant de 4 à 10 atomes de carbone en présence d'un catalyseur, conduisant à la formation d'un mélange réactionnel comprenant l'ester recherché, l'acide et l'alcool non réagis, des sous-produits légers et des sous-produits lourds, qui est soumis à un traitement de purification par des moyens de séparation, afin d'obtenir le (méth)acrylate d'alkyle purifié, ledit traitement de purification étant caractérisé en ce qu'il comprend une étape de déshydratation par séparation membranaire appliqué à l'un au moins des flux suivants : le flux soumis à la distillation finale conduisant à la récupération de l'ester (méth)acrylique purifié, le flux aqueux provenant de la décantation du mélange réactionnel, ou le flux issu de la distillation des sous-produits légers présents dans le mélange réactionnel.

Selon un mode de réalisation, la déshydratation par séparation membranaire est une déshydratation par pervaporation ou par perméation de vapeur.

Selon un mode de réalisation, la séparation membranaire est une séparation sur une membrane inorganique, de préférence zéolithe, et de manière plus particulièrement préférée zéolithe de type T ; ou sur une membrane polymérique hydrophile, de préférence une membrane hydrophile à base d'alcool polyvinylique.

Selon un mode de réalisation, la séparation membranaire est une séparation sur une membrane polymérique hydrophobe telle la membrane 4060 commercialisée par Sulzer.

Selon un mode de réalisation, le procédé est choisi parmi les procédés de type continu, semi-continu ou discontinu.

La présente invention fournit plus particulièrement un procédé de production d'esters (méth)acryliques présentant une bonne sélectivité, permettant une élimination efficace de l'eau et permettant de réduire le coût énergétique tout en minimisant le coût d'installation qui est directement proportionnel pour un procédé membranaire au flux horaire de perméat par mètre carré de membrane installé.

Par ailleurs, dans le cas particulier de l'utilisation de membrane hydrophobe, il est possible de traiter un flux aqueux contenant moins de 7% de composés organiques et ainsi de diminuer la taille de la colonne de purification qui sera placée en aval et donc sa consommation énergétique, voire de la supprimer totalement.

De façon surprenante par rapport au procédé décrit dans le document FR 2 980 475, les étapes de traitement par séparation membranaire selon l'invention ont de très bonnes performances en dehors des domaines de concentration décrits, et permettent de traiter des flux aqueux à des vitesses horaires plus élevées. Ces performances sont liées essentiellement au choix de l'alcool et donc de l'ester mis en oeuvre, notamment en raison de leur caractère apolaire qui rend aisé le passage de l'eau à travers la membrane hydrophile.

BREVE DESCRIPTION DES FIGURES

La **figure 1** représente de manière schématique une installation pour mettre en oeuvre un procédé semi-continu de production d'ester (méth)acrylique.

La **figure 2** représente de manière schématique une installation pour mettre en oeuvre un procédé continu de production d'ester (méth)acrylique avec une catalyse homogène.

La **figure 3** représente de manière schématique une installation pour mettre en oeuvre un procédé continu de production d'ester (méth)acrylique avec une catalyse hétérogène.

DESCRIPTION DE MODES DE REALISATION DE L'INVENTION

L'invention est maintenant décrite plus en détail dans la description qui suit.

Les termes « (méth)acrylique » et « (méth)acrylate » signifient, de façon conventionnelle, « acrylique ou méthacrylique » et « acrylate ou méthacrylate » respectivement.

Sauf mention contraire, les compositions données en pourcentages s'entendent en valeurs massiques.

Le composé alcool utilisé dans le cadre de l'invention peut être linéaire ou ramifié. Il peut s'agir d'un alcool primaire ou d'un alcool secondaire. Il peut comporter 4 ou 5 ou 6 ou 7 ou 8 ou 9 ou 10 atomes de carbone. Il peut être substitué ou non, et de préférence il est non-substitué. Le composé alcool peut notamment être le butanol, le 2-éthylhexanol ou le 2-octanol.

Les esters obtenus correspondants sont l'acrylate de butyle ou le méthacrylate de butyle, l'acrylate de 2-éthylhexyle ou le méthacrylate de 2-éthylhexyle, l'acrylate de 2-octyle ou le méthacrylate de 2-octyle.

De préférence, l'acide (méth)acrylique est l'acide acrylique.

La réaction d'estérification de l'acide (méth)acrylique par l'alcool est effectuée en présence d'un catalyseur qui peut être par exemple une résine échangeuse de cations acides dans le cas d'une catalyse hétérogène, ou dans le cas d'une catalyse homogène, qui peut être un acide inorganique de type acide sulfurique ou un acide organique sulfonique tel que l'acide méthane sulfonique, l'acide para-toluène sulfonique, l'acide benzène sulfonique, l'acide dodécyl sulfonique, ou leurs mélanges.

De manière générale, la réaction d'estérification est effectuée en présence d'un excès stoechiométrique d'alcool.

Le mélange réactionnel (dénommé aussi par la suite flux de réaction) contient l'ester produit ainsi que les réactifs n'ayant pas réagi - principalement l'alcool - qui représentent les composés légers à séparer et si possible à recycler, et des sous-produits lourds issus de réactions secondaires.

Selon l'invention, les flux soumis à la déshydratation par séparation membranaire sont des flux aqueux exempts ou quasi exempts de sous-produits lourds, c'est-à-dire des flux en aval de traitement de séparation des sous-produits présentant une température d'ébullition supérieure à celle de l'ester (méth)acrylique.

Selon un mode de réalisation, le flux traité par séparation membranaire est un flux soumis à la distillation finale conduisant à la récupération de l'ester (méth)acrylique purifié. Selon ce mode de réalisation, le flux aqueux contient par conséquent une teneur importante en ester (méth)acrylique, en général supérieure à 50%.

Selon un mode de réalisation, le flux traité par séparation membranaire est un flux aqueux généré par décantation du mélange réactionnel, la phase organique séparée par décantation étant elle quasi exempte d'eau. Selon ce mode de réalisation, le flux aqueux contient plus de 80% d'eau et une faible teneur en composés organiques, en général inférieure à 10%.

Selon un mode de réalisation, le flux traité par séparation membranaire est un flux issu de la distillation des sous-produits légers présents dans le mélange réactionnel. Selon ce mode de réalisation, le flux aqueux contient par conséquent une teneur importante en alcool, en général supérieure à 20%.

Selon l'invention, les flux traités par séparation membranaire peuvent contenir de l'eau dans une large gamme, de 0,5 à 99%, de l'alcool dans une gamme de concentration allant d'environ 1 à 60%, de l'ester dans une gamme de concentration allant d'environ 0,5 à 70%, et dans une moindre mesure de l'acide dans une gamme de concentration pouvant aller jusqu'à 15%. Le flux soumis à la distillation finale conduisant à la récupération de l'ester (méth)acrylique purifié est le flux comprenant 50 à 70 % d'ester (méth)acrylique, de 20 à 30 % d'alcool, de 0 à 12 % d'acide (méth)acrylique, et de 3 à 12 % d'eau. Le flux aqueux provenant de la décantation du mélange réactionnel est le flux comprenant 80 à 99 % d'eau, moins de 2 % d'ester (méth)acrylique et de 1 à 20 % d'alcool. Le flux de tête issu de la distillation du mélange réactionnel est le flux comprenant 0,5 à 15 % d'acide (méth)acrylique, de 25 à 65 % d'ester (méth)acrylique, de 20 à 60 % d'alcool et de 0,5 à 15 % d'eau.

Selon l'invention, l'étape de déshydratation par séparation membranaire est réalisée par pervaporation (charge en phase liquide et vaporisation du perméat à la traversée de la membrane), ou par perméation de vapeur (charge en phase vapeur), de préférence à une température allant de 50°C à 100°C, plus préférentiellement de 55°C à 85°C.

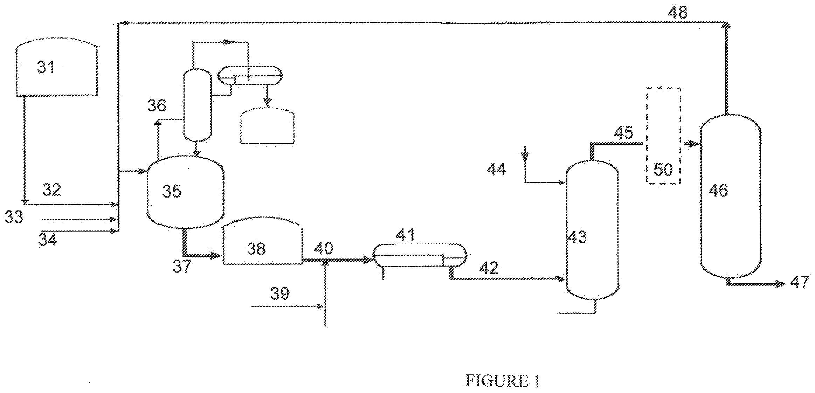

La **figure 1** illustre le mode de réalisation selon lequel l'étape de séparation membranaire est appliquée à un flux soumis à la distillation finale conduisant à la récupération de l'ester (méth)acrylique purifié. La **figure 1** correspond à un procédé semi-continu (avec réaction en « batch »), l'installation comportant un unique réacteur 35. Ce réacteur 35 est alimenté par une ligne d'amenée de composé alcool 32 (issue d'un réservoir de composé alcool 31), une ligne d'amenée d'acide (méth)acrylique 33 et une ligne d'amenée d'additif(s) 34, par exemple une ligne d'amenée de catalyseur acide tel que l'acide sulfurique et/ou d'inhibiteur de polymérisation.

Le réacteur 35 est pourvu d'un système d'élimination d'eau 36 comportant une colonne, un décanteur, une ligne de recyclage, une ligne de purge d'eau.

En sortie du réacteur 35 est récupéré le flux de réaction par une ligne de soutirage de flux de réaction 37. Celle-ci alimente un bac de stockage 38 en sortie duquel le flux de réaction est récupéré par une première ligne de transfert 40. Une ligne d'amenée de composé neutralisant 39 est connectée sur la première ligne de transfert 40 et permet la neutralisation du flux de réaction. A titre de composé neutralisant, on utilise un composé basique tel que la soude.

La première ligne de transfert 40 alimente un décanteur 41 permettant de purger une partie de l'eau contenue dans le flux de réaction 40. En sortie du décanteur 41 est connectée une deuxième ligne de transfert 42 qui alimente une colonne de lavage 43 pourvue d'une alimentation en eau 44. Une troisième ligne de transfert 45 est connectée en sortie de la colonne de lavage 43, qui alimente une colonne de distillation 46, après une élimination d'eau effectuée au moyen d'une unité de déshydratation par séparation membranaire 50 disposée sur la ligne de transfert 45.

En pied de la colonne de distillation 46 est connectée une ligne de soutirage de flux riche en composé ester (méth)acrylique 47, par laquelle est récupéré le composé ester (méth) acrylique essentiellement purifié.

En tête de la colonne de distillation 46 est connectée une ligne de recyclage 48 de flux riche en réactifs non réagis, en particulier riche en composé alcool non réagi, par laquelle est récupéré un mélange de composé alcool, acide, de composé ester (méth)acrylique et d'eau. Ce mélange est renvoyé à la réaction, la ligne de recyclage de flux riche en composé alcool non réagi 48 étant connectée en entrée du réacteur 35, après une éventuelle élimination supplémentaire de l'eau contenue dans le mélange.

Cette élimination supplémentaire peut être effectuée au moyen d'une unité de déshydratation par séparation membranaire (non représentée) disposée sur la ligne de recyclage de flux riche en composé alcool non réagi 48.

L'unité de déshydratation par séparation membranaire 50 peut être une unité de pervaporation (charge en phase liquide et vaporisation du perméat à la traversée de la membrane), ou une unité de perméation de vapeur (charge en phase vapeur).

La déshydratation est de préférence mise en oeuvre à une température allant de 50°C à 100°C, plus préférentiellement de 55°C à 85°C.

Les membranes utilisées sont de préférence des membranes hydrophiles à base d'alcool polyvinylique ou une membrane inorganique céramique comme une zéolithe.

Le mélange qui est déshydraté dans l'unité de déshydratation par séparation membranaire 50 comprend, de 50 à 70 % d'ester (méth)acrylique, de 20 à 30 % d'alcool, de 0 à 12 % d'acide (méth)acrylique, et de 3 à 12 % d'eau.

L'installation représentée à la **figure 1** peut en particulier être utilisée pour la production en semi continu d'acrylate de butyle à partir de butanol, d'acrylate de 2-éthylhexyle à partir de 2-éthyl hexanol, ou d'acrylate de 2-octyle à partir de 2-octanol.

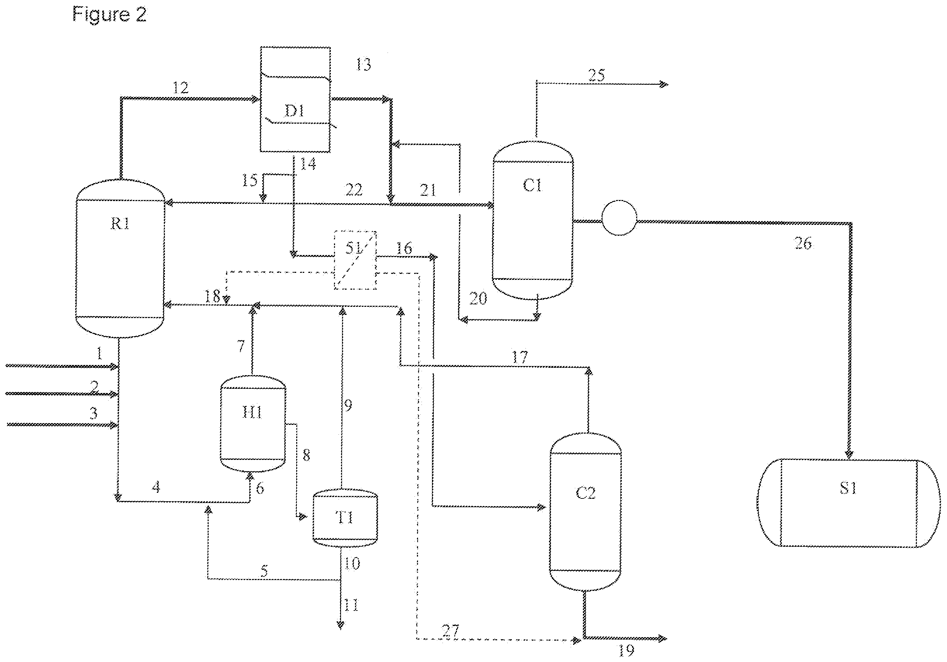

La **figure 2** illustre un mode de réalisation selon lequel l'étape de séparation membranaire est appliquée au flux aqueux généré par décantation du mélange réactionnel.

Selon la **figure 2,** illustrant un procédé continu, l'installation comporte un unique réacteur R1. Ce réacteur R1 est alimenté par une ligne d'amenée de composé alcool 1, une ligne d'amenée d'acide (méth)acrylique 2 et une ligne d'amenée d'additif(s) 3 par exemple une ligne d'amenée de catalyseur tel que acide sulfurique, acide méthane sulfonique ou acide para toluène sulfonique.

Le réacteur R1 est relié à une colonne (non représentée), un décanteur D1, une ligne de recyclage.

En sortie du réacteur et de sa colonne, les produits de tête sont envoyés via la ligne 12 vers un décanteur D1.

La phase organique est soutirée ligne 13 pour être retournée soit vers le réacteur ligne 22 soit vers une colonne de distillation C1 via la ligne 21. Le produit final après distillation dans la colonne C1 étant envoyé vers S1 via une ligne de soutirage latéral 26.

Le produit de pied de colonne C1 comprenant essentiellement l'ester formé et des produits lourds est recyclé à la réaction via la ligne 20 tandis que les produits légers sont envoyés vers un système de traitement de purges via la ligne 25.

La phase aqueuse du décanteur D1 est envoyée via la ligne 14, soit au reflux de la colonne du réacteur via 15, soit vers une colonne de distillation C2 via 16. La colonne C2 permet de récupérer les composés organiques contenus dans cette phase aqueuse et de les renvoyer via la ligne 17 vers le réacteur.

Selon l'invention, une unité de déshydratation par séparation membranaire 51 est placée en sortie du décanteur D1 sur la ligne 14 de la phase aqueuse. On peut ensuite envoyer la phase organique contenue dans cette phase riche en eau séparée par ladite unité de déshydratation vers le réacteur via la ligne 18, et la phase aqueuse exempte de composés organiques, soit vers la colonne C2 via la ligne 16, soit directement vers la station de traitement des eaux en fonction du nombre d'étages de séparation du procédé membranaire via la ligne 27.

L'apport calorique du réacteur R1 est assurée par la boucle de circulation 4 qui est réchauffée au niveau du bouilleur H1 puis renvoyée dans le réacteur via la ligne 7, ou envoyée vers un système de traitement thermique via la ligne 8 tel un évaporateur à film ou un craqueur thermique de façon à régénérer une partie du flux pour le renvoyer vers le réacteur via la ligne 9. Le flux traité 10 (produits lourds) est purgé via la ligne 11 ou recyclé partiellement via la ligne 5.

L'unité de déshydratation par séparation membranaire 51 peut être telle que décrite ci-dessus en relation avec la **figure 1,** mais avec de préférence une membrane hydrophobe, telle que telle la membrane 4060 commercialisée par Sulzer.

Dans ce mode de réalisation, le flux qui est soumis à la déshydratation par séparation membranaire 51 est un flux riche en eau, comprenant de 80 à 99% d'eau, moins de 2 % d'ester (méth)acrylique et de 1 à 20 % d'alcool.

L'installation représentée à la **figure 2** peut en particulier être utilisée pour la production en continu d'acrylate de butyle à partir de butanol et d'acide acrylique en catalyse acide homogène.

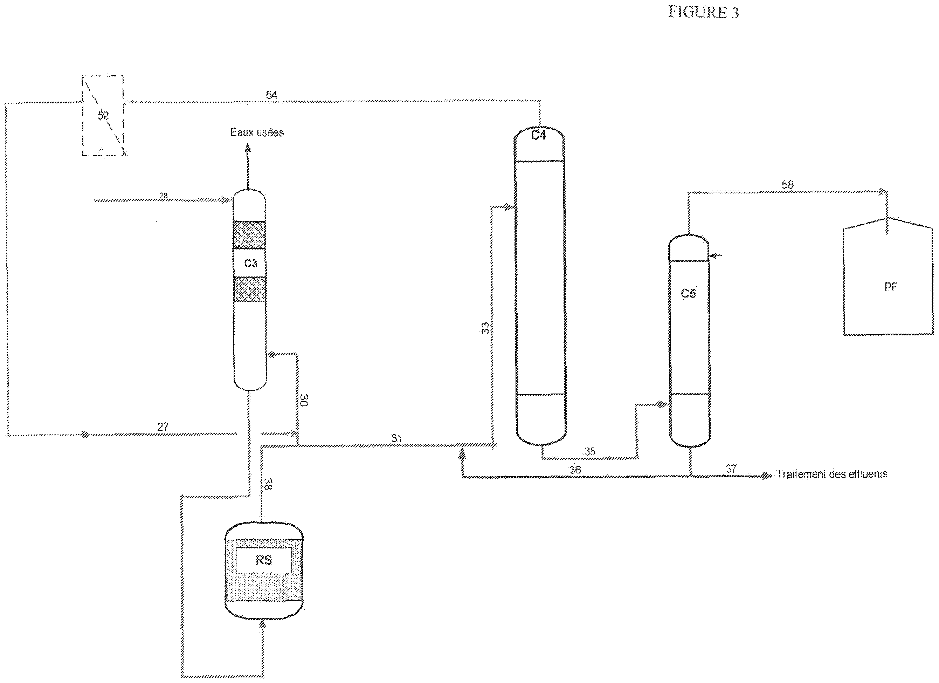

La **figure 3** illustre un mode de réalisation selon lequel l'étape de séparation membranaire est appliquée sur un flux issu de la distillation des sous-produits légers présents dans le mélange réactionnel.

En faisant référence à la **figure 3,** selon un autre mode de réalisation d'un procédé continu, l'installation comporte un unique réacteur RS. Ce réacteur RS contient une résine acide comme catalyseur hétérogène. Il est surmonté d'une colonne de distillation C3 qui permet d'éliminer l'eau de réaction vers la station de traitement des eaux usées. La colonne C3 est alimentée en tête par un flux d'alcool frais 28. Le pied de la colonne C3 est envoyé sur le réacteur RS. L'acide acrylique ou méthacrylique utilisé dans la réaction est injecté via la ligne 27. Le produit en tête du réacteur RS retourne en pied de colonne via la ligne 38 ainsi qu'en partie vers une colonne de distillation C4.. L'alcool recyclé provenant de la tête de colonne C4 via la ligne 54 est envoyé dans le réacteur via la ligne 27.

Selon l'invention, le flux de tête de colonne C4 est soumis à une étape de déshydratation dans une unité de séparation membranaire 52 qui permet d'éliminer une grande partie de l'eau contenue dans le flux avant recyclage à la réaction. Le flux de pied de colonne C4 est envoyé via la ligne 35 vers la colonne finale d'équeutage C5 qui permet de séparer en pied les sous-produits lourds, et en tête l'ester recherché. L'ester purifié est envoyé dans le bac de stockage PF via la ligne 58. Le flux de pied de colonne C5 est, soit partiellement recyclé à l'alimentation de la colonne C4, soit envoyé vers une station de traitement d'effluents.

L'unité de déshydratation par séparation membranaire 52 peut être telle que décrite ci-dessus en relation avec la **figure 1.**

Selon ce mode de réalisation, le flux qui est soumis à l'unité de déshydratation par séparation membranaire 52 comprend de 0,5 à 15 % d'acide (méth)acrylique, de 25 à 65 % d'ester (méth)acrylique, de 20 à 60 % d'alcool et de 0,5 à 15 % d'eau.

L'installation représentée à la **figure 3** peut en particulier être utilisée pour la production en continu d'acrylate de 2-éthylhexyle à partir de 2-éthyl hexanol et d'acide acrylique en catalyse hétérogène acide.

Les exemples suivants illustrent la présente invention.

EXEMPLES

Exemple 1

Des essais ont été réalisés sur un pilote de séparation membranaire Sulzer, présentant une surface de membrane de 170 cm$^2$. La membrane testée est une membrane hydrophile à base d'alcool polyvinylique de référence Pervap® 1201.

Différents flux issus d'une installation réelle de production d'acrylate de butyle ont été appliqués sur la membrane. Ces flux correspondent au flux 45 représenté sur la **figure 1.** Les flux comprennent principalement de l'acrylate de butyle, du butanol, de l'eau, et éventuellement de l'acide acrylique.

Le perméat obtenu contient entre 95% et 99 % d'eau. En d'autres termes, la sélectivité de la membrane est excellente, et celle-ci n'est pas sensiblement affectée par la présence de l'ester.

En fonction des températures et des conditions d'essai, le flux traversant la membrane varie de 0,8 kg/m$^2$.h à 2,7 kg/m$^2$.h, ce qui représente des valeurs élevées par rapport aux valeurs généralement atteintes dans les procédés de l'art antérieur.

Aucune dégradation de la membrane n'a été observée malgré la présence d'acide acrylique et d'ester acrylique.

Les résultats obtenus avec la membrane Pervap® 1201 sont résumés dans le tableau 1.

Tableau 1

| | | Pourcentage massique Alimentation | | | | P aval | Pourcentage massique Perméat | | | | Flux de perméat | Sélectivité | |
|---|---|---|---|---|---|---|---|---|---|---|---|---|---|
| | T (°C) | BuOH | ABU | H2O | AA | Mbar | BuOH | ABU | H2O | AA | J kg/m$^2$.h | $\alpha$ H2O/R | $\beta$ H2Of/H2Oi |
| A | 65,0 | 26,9 | 56,4 | 5,5 | 11,1 | 60,0 | 0,7 | 0,2 | 98,4 | 0,6 | 0,8 | 1081 | 18,0 |
| | 75,0 | 26,9 | 56,4 | 5,5 | 11,1 | 60,0 | 0,6 | 0,1 | 98,1 | 1,1 | 1,3 | 901 | 18,0 |
| | 80,0 | 26,9 | 56,4 | 5,5 | 11,1 | 60,0 | 0,6 | 0,2 | 98,5 | 0,7 | 1,7 | 1115 | 18,0 |
| | 90,0 | 26,9 | 56,4 | 5,5 | 11,1 | 60,0 | 0,3 | 0,1 | 99,2 | 0,4 | 1,8 | 2235 | 18,2 |
| B | 62,8 | 30,0 | 66,7 | 3,3 | 0,0 | 60,0 | 4,1 | 0,1 | 95,6 | 0,0 | 0,9 | 638 | 29,3 |
| | 69,0 | 30,0 | 66,7 | 3,3 | 0,0 | 60,0 | 2,9 | 0,2 | 96,8 | 0,0 | 1,3 | 909 | 29,7 |
| | 80,0 | 30,0 | 66,7 | 3,3 | 0,0 | 60,0 | 1,9 | 0,2 | 97,9 | 0,0 | 2,0 | 1401 | 30,0 |
| | 87,2 | 30,0 | 66,7 | 3,3 | 0,0 | 60,0 | 1,4 | 0,1 | 98,5 | 0,0 | 2,7 | 1922 | 30,2 |

Le facteur de séparation $\alpha$ est calculé par :

$$\alpha_{\frac{H2O}{R}} = \frac{y_{H2O}/y_R}{x_{H2O}/x_R}$$

avec $y_{H2O}$ et $x_{H2O}$ sont les fractions massiques de l'eau dans le perméat et dans l'alimentation, respectivement. Dans le cas de pervaporation des mélanges multiconstituants, $y_R$ et $x_R$ sont (1- $y_{H2O}$) et (1- $x_{H2O}$), respectivement.

Le facteur $\beta$ ou facteur d'enrichissement est le rapport entre la concentration d'eau dans le perméat et la concentration en eau de l'alimentation

Exemple 2

Des essais ont été réalisés sur un pilote de séparation membranaire Sulzer, présentant une surface de membrane de 170 cm$^2$. La membrane testée est une membrane hydrophobe de référence Pervap® 4060.

Un flux issu d'une installation réelle de production d'acrylate de butyle a été appliqué sur la membrane. Ce flux correspond au flux 14 représenté sur la **figure 2.** Ce flux comprend principalement de l'eau (95,2%) avec un peu de butanol (4,7%) et des traces d'acrylate de butyle (0,1%).

Les résultats obtenus à différentes températures avec la membrane Pervap® 4060 sont résumés dans le tableau 2.

Tableau 2

| | Pourcentage massique | | | P aval | Pourcentage massique | | | Flux de perméat | Sélectivité | Sélectivité |
|---|---|---|---|---|---|---|---|---|---|---|
| | Alimentation | | | | Perméat | | | | | |
| T (°C) | BuOH | ABU | $H_2O$ | mbar | BuOH | ABU | $H_2O$ | J (kg/m²·h) | $\alpha_{H2O/R}$ | $\beta_{H2Of/H2Oi}$ |
| 59,75 | 4,7 | 0,1 | 95,2 | 60 | 76,3 | 8,0 | 15,2 | 1,86 | 111 | 17,6 |
| 67,15 | 4,7 | 0,1 | 95,2 | 60 | 76,2 | 8,0 | 15,2 | 3,26 | 111 | 17,6 |
| 78,4 | 4,7 | 0,1 | 95,2 | 60 | 77,3 | 5,4 | 16,0 | 6,06 | 105 | 17,3 |
| 90,65 | 4,7 | 0,1 | 95,2 | 60 | 78,9 | 3,1 | 16,9 | 7,10 | 98 | 17,1 |

Le perméat enrichi en alcool peut être avantageusement recyclé à la réaction.

Exemple 3

Des essais ont été réalisés sur un pilote de séparation membranaire Sulzer, présentant une surface de membrane de 170 cm². La membrane testée est une membrane hydrophile à base d'alcool polyvinylique de référence Pervap® 1201.
Deux flux issus d'une installation réelle de production d'acrylate de 2-éthylhexyle (AE2H) à partir de 2-éthyl hexanol (2EHeOH) ont été appliqués sur la membrane à différentes températures. Ces flux correspondent au flux 54 représenté sur la **figure 3.** Les flux comprennent de l'alcool, de l'ester, de l'acide et un peu d'eau.
Les résultats obtenus avec la membrane Pervap® 1201 sont résumés dans le tableau 3.

Tableau 3

| T (°C) | Pourcentage massique — Alimentation | | | | P aval | Pourcentage massique — Perméat | | | | Flux de perméat | Sélectivité | Sélectivité |
|---|---|---|---|---|---|---|---|---|---|---|---|---|
| | 2EHeOH | AE2H | $H_2O$ | AA | mbar | 2EHeOH | AE2H | $H_2O$ | AA | J (kg/m$^2$.h) | $\alpha_{H2O/R}$ | $\beta_{H2Of/H2Oi}$ |
| 61,6 | 31,9 | 55,5 | 2,2 | 10,1 | 60 | 0,0 | 0,0 | 96,3 | 3,4 | 1,01 | 1134 | 43,5 |
| 69,75 | 31,9 | 55,5 | 2,2 | 10,1 | 60 | 0,0 | 0,0 | 95,1 | 4,6 | 1,46 | 848 | 42,9 |
| 78,8 | 31,9 | 55,5 | 2,2 | 10,1 | 60 | 0,0 | 0,0 | 95,0 | 4,7 | 2,17 | 832 | 42,9 |
| 85,5 | 31,9 | 55,5 | 2,2 | 10,1 | 60 | 0,0 | 0,0 | 95,0 | 4,8 | 2,55 | 830 | 42,9 |
| 60,8 | 52,8 | 32,5 | 2,1 | 12,3 | 60 | 0,1 | 0,0 | 96,3 | 3,3 | 0,79 | 1213 | 45,4 |
| 69,05 | 52,8 | 32,5 | 2,1 | 12,3 | 60 | 0,1 | 0,0 | 95,7 | 4,0 | 1,24 | 1027 | 45,1 |
| 79,95 | 52,8 | 32,5 | 2,1 | 12,3 | 60 | 0,0 | 0,0 | 95,7 | 4,3 | 1,75 | 1015 | 45,0 |
| 87,75 | 52,8 | 32,5 | 2,1 | 12,3 | 60 | 0,1 | 0,0 | 96,4 | 3,3 | 2,12 | 1224 | 45,4 |

**EP 3 079 797 B1**

L'unité de déshydratation permet d'éliminer la majorité de l'eau présente dans le flux contenant le 2-éthyl hexanol résiduel avant son recyclage à la réaction.

**Revendications**

1. Procédé de production d'un (méth)acrylate d'alkyle comportant une chaine alkyle linéaire ou ramifiée comportant de 4 à 10 atomes de carbone, par estérification directe de l'acide (méth)acrylique avec un alcool linéaire ou ramifié comportant de 4 à 10 atomes de carbone en présence d'un catalyseur, conduisant à la formation d'un mélange réactionnel comprenant l'ester recherché, l'acide et l'alcool non réagis, de l'eau, des sous-produits légers et des sous-produits lourds, qui est soumis à un traitement de purification par des moyens de séparation, afin d'obtenir le (méth)acrylate d'alkyle purifié,
ledit traitement de purification étant **caractérisé en ce qu'**il comprend une étape de déshydratation par séparation membranaire appliqué à l'un au moins des flux suivants :

- le flux soumis à la distillation finale conduisant à la récupération de l'ester (méth)acrylique purifié, ledit flux comprenant de 50 à 70 % massique d'ester (méth)acrylique, de 20 à 30 % massique d'alcool, de 0 à 12 % massique d'acide (méth)acrylique, et de 3 à 12 % massique d'eau ;
- le flux aqueux provenant de la décantation du mélange réactionnel, ledit flux comprenant de 80 à 99% massique d'eau, moins de 2 % massique d'ester (méth)acrylique et de 1 à 20 % massique d'alcool ;
- le flux de tête issu de la distillation du mélange réactionnel, ledit flux comprenant de 0,5 à 15 % massique d'acide (méth)acrylique, de 25 à 65 % massique d'ester (méth)acrylique, de 20 à 60 % massique d'alcool et de 0,5 à 15 % massique d'eau.

2. Procédé selon la revendication 1 **caractérisé en ce que** l'alcool est le butanol, le 2-éthylhexanol ou le 2-octanol.

3. Procédé selon la revendication 1 ou 2 caractérisé en ce l'acide est l'acide acrylique.

4. Procédé selon l'une quelconque des revendications précédentes **caractérisé en ce que** la déshydratation par séparation membranaire est une déshydratation par pervaporation ou par perméation de vapeur.

5. Procédé selon l'une quelconque des revendications précédentes **caractérisé en ce que** la séparation membranaire est une séparation sur une membrane inorganique, sur une membrane polymérique hydrophile, ou une séparation sur une membrane polymérique hydrophobe.

6. Procédé selon la revendication 5 **caractérisé en ce que** la séparation membranaire est réalisée sur une zéolithe ou une membrane hydrophile à base d'alcool polyvinylique.

7. Procédé selon l'une quelconque des revendications précédentes **caractérisé en ce qu'**il est de type continu, semi-continu ou discontinu.

8. Procédé selon l'une quelconque des revendications précédentes, **caractérisé en ce que** l'étape de déshydratation par séparation membranaire est appliquée au flux soumis à la distillation finale conduisant à la récupération de l'ester (méth)acrylique purifié, dans un procédé de production d'acrylate de butyle.

9. Procédé selon l'une quelconque des revendications 1 à 7, **caractérisé en ce que** l'étape de déshydratation par séparation membranaire est appliquée au flux aqueux provenant de la décantation du mélange réactionnel, dans un procédé de production en continu d'acrylate de butyle en catalyse homogène.

10. Procédé selon l'une quelconque des revendications 1 à 7, **caractérisé en ce que** l'étape de déshydratation par séparation membranaire est appliquée au flux de tête issu de la distillation du mélange réactionnel, dans un procédé de production en continu d'acrylate de 2-éthylhexyle en catalyse hétérogène acide.

**Patentansprüche**

1. Verfahren zur Herstellung eines Alkyl(meth)-acrylats mit einer linearen oder verzweigten Alkylkette mit 4 bis 10 Kohlenstoffatomen durch Direktveresterung von (Meth)acrylsäure mit einem linearen oder verzweigten Alkohol mit 4 bis 10 Kohlenstoffatomen in Gegenwart eines Katalysators, was zur Bildung einer Reaktionsmischung führt, die den gewünschten Ester, nicht umgesetzte Säure und nicht umgesetzten Alkohol, Wasser, leichte Nebenprodukte und schwere Nebenprodukte umfasst und die einer Reinigungsbehandlung durch Trennmittel zum Erhalt von gereinigtem Alkyl-(meth)acrylat unterworfen wird,

wobei die Reinigungsbehandlung **dadurch gekennzeichnet ist, dass** sie einen Schritt der Dehydratisierung durch Membrantrennung umfasst, der auf mindestens einen der folgenden Ströme angewendet wird:

- den der abschließenden Destillation, die zur Gewinnung des gereinigten(Meth)acrylsäureesters führt, unterworfenen Strom, wobei der Strom 50 bis 70 Massen-% (Meth)acrylsäureester, 20 bis 30 Massen-% Alkohol, 0 bis 12 Massen-% (Meth)acrylsäure und 3 bis 12 Massen-% Wasser umfasst;
- den wässrigen Strom aus dem Absetzenlassen der Reaktionsmischung, wobei der Strom 80 bis 99 Massen-% Wasser, weniger als 2 Massen-% (Meth)-acrylsäureester und 1 bis 20 Massen-% Alkohol umfasst;
- den Kopfstrom aus der Destillation der Reaktionsmischung, wobei der Strom 0,5 bis 15 Massen-% (Meth)acrylsäure, 25 bis 65 Massen-% (Meth)acrylsäureester, 20 bis 60 Massen-% Alkohol und 0,5 bis 15 Massen-% Wasser umfasst.

2. Verfahren nach Anspruch 1, **dadurch gekennzeichnet, dass** es sich bei dem Alkohol um Butanol, 2-Ethylhexanol oder 2-Octanol handelt.

3. Verfahren nach Anspruch 1 oder 2, **dadurch gekennzeichnet, dass** es sich bei der Säure um Acrylsäure handelt.

4. Verfahren nach einem der vorhergehenden Ansprüche, **dadurch gekennzeichnet, dass** es sich bei der Dehydratisierung durch Membrantrennung um eine Dehydratisierung durch Pervaporation oder Dampfpermeation handelt.

5. Verfahren nach einem der vorhergehenden Ansprüche, **dadurch gekennzeichnet, dass** es sich bei der Membrantrennung um eine Trennung an einer anorganischen Membran, an einer hydrophilen Polymermembran oder eine Trennung an einer hydrophoben Polymermembran handelt.

6. Verfahren nach Anspruch 5, **dadurch gekennzeichnet, dass** die Membrantrennung an einem Zeolith oder einer hydrophilen Membran auf Basis von Polyvinylalkohol durchgeführt wird.

7. Verfahren nach einem der vorhergehenden Ansprüche, **dadurch gekennzeichnet, dass** es vom kontinuierlichen, halbkontinuierlichen oder diskontinuierlichen Typ ist.

8. Verfahren nach einem der vorhergehenden Ansprüche, **dadurch gekennzeichnet, dass** der Schritt der Dehydratisierung durch Membrantrennung auf den der abschließenden Destillation, die zur Gewinnung des gereinigten(Meth)acrylsäureesters führt, unterworfenen Strom bei der Herstellung von Butylacrylat angewendet wird.

9. Verfahren nach einem der Ansprüche 1 bis 7, **dadurch gekennzeichnet, dass** der Schritt der Dehydratisierung durch Membrantrennung auf den wässrigen Strom aus dem Absetzenlassen der Reaktionsmischung bei einem Verfahren zur kontinuierlichen Herstellung von Butylacrylat unter homogener Katalyse angewendet wird.

10. Verfahren nach einem der Ansprüche 1 bis 7, **dadurch gekennzeichnet, dass** der Schritt der Dehydratisierung durch Membrantrennung auf den Kopfstrom aus der Destillation der Reaktionsmischung bei einem Verfahren zur kontinuierlichen Herstellung von 2-Ethylhexylacrylat unter heterogener saurer Katalyse angewendet wird.

**Claims**

1. Process for producing an alkyl (meth)acrylate comprising a linear or branched alkyl chain comprising from 4 to 10 carbon atoms, by direct esterification of (meth)acrylic acid with a linear or branched alcohol comprising from 4 to 10 carbon atoms in the presence of a catalyst, leading to the formation of a reaction mixture comprising the desired ester, the unreacted acid and alcohol, water, light by-products and heavy by-products, which mixture undergoes a treatment for purification by separation means, in order to obtain the purified alkyl (meth)acrylate,

said purification treatment being **characterized in that** it comprises a step of membrane separation dehydration applied to at least one of the following streams:

- the stream subjected to the final distillation leading to the recovery of the purified (meth)acrylic ester, said streams comprising from 50 to 70% by weight of (meth) acrylic ester, from 20 to 30% by weight of alcohol, from 0 to 12% by weight of (meth)acrylic acid, and from 3 to 12% by weight of water;

- the aqueous stream originating from the settling out of the reaction mixture, said streams comprising from 80 to 99% by weight of water, less than 2% by weight of (meth) acrylic ester and from 1 to 20% by weight of alcohol;

- the top stream resulting from the distillation of the reaction mixture, said stream comprising from 0.5 to 15% by weight of (meth)acrylic acid, from 25 to 65% by weight of (meth)acrylic ester, from 20 to 60% by weight of alcohol and from 0.5 to 15% by weight of water.

2. Process according to Claim 1, **characterized in that** the alcohol is butanol, 2-ethylhexanol or 2-octanol.

3. Process according to Claim 1 or 2, **characterized in that** the acid is acrylic acid.

4. Process according to any one of the preceding claims, **characterized in that** the membrane separation dehydration is a dehydration by pervaporation or by vapor permeation.

5. Process according to any one of the preceding claims, **characterized in that** the membrane separation is a separation on an inorganic membrane, on a hydrophilic polymeric membrane, or a separation on a hydrophobic polymeric membrane.

6. Process according to Claim 5, **characterized in that** the membrane separation is carried out on a zeolite or a hydrophilic membrane based on polyvinyl alcohol.

7. Process according to any one of the preceding claims, **characterized in that** it is of continuous, semi-continuous, or batchwise type.

8. Process according to any one of the preceding claims, **characterized in that** the step of membrane separation dehydration is applied to the stream subjected to the final distillation leading to the recovery of the purified (meth)acrylic ester, in a process for producing butyl acrylate.

9. Process according to any one of Claims 1 to 7, **characterized in that** the step of membrane separation dehydration is applied to the aqueous stream originating from the settling out of the reaction mixture, in a process for the continuous production of butyl acrylate in homogeneous catalysis.

10. Process according to any one of Claims 1 to 7, **characterized in that** the step of membrane separation dehydration is applied to the top stream resulting from the distillation of the reaction mixture, in a process for the continuous production of 2-ethylhexyl acrylate in heterogeneous acid catalysis.

FIGURE 1

EP 3 079 797 B1

Figure 2

EP 3 079 797 B1

FIGURE 3

RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2980475